(19) Europäisches Patentamt
European Patent Office
Office européen des brevets

(11) Veröffentlichungsnummer: **0 070 443**
**A1**

(12) # EUROPÄISCHE PATENTANMELDUNG

(21) Anmeldenummer: **82105969.8**

(22) Anmeldetag: **05.07.82**

(51) Int. Cl.³: **C 07 F 9/40**, A 01 N 57/22

(30) Priorität: **16.07.81 DE 3128103**

(43) Veröffentlichungstag der Anmeldung: **26.01.83**
**Patentblatt 83/4**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Hoffmann, Hellmut, Prof. Dr., Tersteegenweg 17, D-5600 Wuppertal 1 (DE)**
Erfinder: **Hammann, Ingeborg, Dr., Belfortstrasse 9, D-5000 Koeln 1 (DE)**
Erfinder: **Homeyer, Bernhard, Dr., Obere Strasse 28, D-5090 Leverkusen 3 (DE)**

(54) **Thionophosphonsäureester, Verfahren zu ihrer Herstellung und ihre Verwendung als Schädlingsbekämpfungsmittel.**

(57) Die Erfindung betrifft neue Thionophosphonsäureester der Formel I

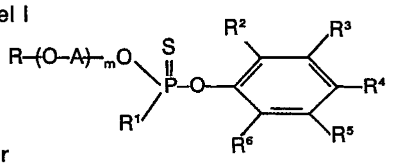
(I)

in welcher

R für gegebenenfalls substituiertes geradkettiges oder verzweigtes Alkyl steht,

A für geradkettiges oder verzweigtes Alkandiyl (Alkylen) steht,

m für 1, 2 oder 3 steht,

$R^1$ für gegebenenfalls substituiertes geradkettiges oder verzweigtes Alkyl steht,

$R^2$ für Wasserstoff, Halogen oder Nitro steht,

$R^3$ für Wasserstoff, Halogen, Nitro oder gegebenenfalls substituiertes Alkyl steht,

$R^4$ für Wasserstoff, Halogen, gegebenenfalls substituiertes Alkyl steht, und – für den Fall, dass $R^1$ für Methyl steht – auch für Nitro oder den Rest $-S(O)_nR^7$ steht, worin n für Null, 1 oder 2 steht und $R^7$ für gegebenenfalls substituiertes Alkyl steht, und

$R^5$ und $R^6$ unabhängig voneinander für Wasserstoff und – für den Fall, dass $R^4$ für Halogen steht – auch für Halogen stehen können,

mit der Maßgabe, daß wenigstens einer der Substituenten $R^2$, $R^3$, $R^4$, $R^5$ und $R^6$ von Wasserstoff verschieden ist, welche dadurch erhalten werden, daß man Thonophosphonsäureesterhalogenide der Formel II

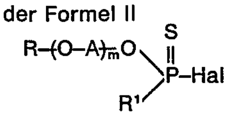
(II)

in welcher

R, A, m und $R^1$ die angegebene Bedeutung haben und Hal für Halogen steht,

mit Phenolderivate der Formel III

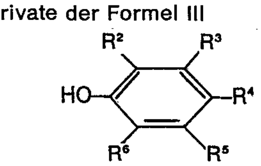
(III)

in welcher

$R^2$, $R^3$, $R^4$, $R^5$ und $R^6$ die oben angegebene Bedeutung haben,

oder mit deren Salzen gegebenenfalls in Gegenwart von Säurebindemitteln und gegebenenfalls in Gegenwart von Verdünnungsmitteln umsetzt.

Die erfindungsgemäßen Thionophosphonsäureester der Formel (I) können als Schädlingsbekämpfungsmittel, insbesondere als Insektizide, Akarizide und Nematizide verwendet werden.

EP 0 070 443 A1

0070443

BAYER AKTIENGESELLSCHAFT 5090 Leverkusen, Bayerwerk

Zentralbereich
Patente, Marken und Lizenzen S/ABc
Ia

Thionophosphonsäureester, Verfahren zu ihrer Herstellung und ihre Verwendung als Schädlingsbekämpfungsmittel

Die Erfindung betrifft neue Thionophosphonsäureester, ein Verfahren zu ihrer Herstellung, sie enthaltende Schädlingsbekämpfungsmittel, ihre Herstellung und Verwendung als Schädlingsbekämpfungsmittel, insbesondere als Insektizide, Akarizide und Nematizide.

Es ist bekannt, daß bestimmte Thionophosphorsäureester, wie z.B. O,O-Dimethyl-O-(4-nitro-phenyl)-thionophosphorsäureester (Methyl-Parathion) und O,O-Dimethyl-O-(3-methyl-4-methylthio-phenyl)-thionophosphorsäureester (Fenthion) zur Schädlingsbekämpfung verwendet werden können. Die genannten Verbindungen stellen anerkannt gute und in der Praxis bewährte Handelsprodukte dar.

Es wurden nun neue O-Alkoxyalkyl-thionophosphonsäureester der Formel I

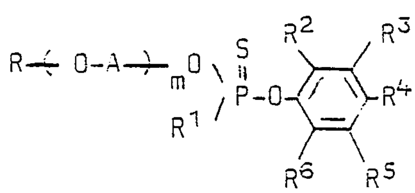

(I)

0070443

gefunden, in welcher

R für gegebenenfalls substituiertes geradkettiges oder verzweigtes Alkyl steht,

A für geradkettiges oder verzweigtes Alkandiyl (Alkylen) steht,

m für 1, 2 oder 3 steht,

$R^1$ für gegebenenfalls substituiertes geradkettiges oder verzweigtes Alkyl steht,

$R^2$ für Wasserstoff, Halogen oder Nitro steht,

$R^3$ für Wasserstoff, Halogen, Nitro oder gegebenenfalls substituiertes Alkyl steht,

$R^4$ für Wasserstoff, Halogen, gegebenenfalls substituiertes Alkyl steht, und - für den Fall, daß $R^1$ für Methyl steht - auch für Nitro oder den Rest $-S(O)_nR^7$ steht, worin n für Null, 1 oder 2 steht und $R^7$ für gegebenenfalls substituiertes Alkyl steht und

$R^5$ und $R^6$ unabhängig voneinander für Wasserstoff und - für den Fall daß $R^4$ für Halogen steht - auch für Halogen stehen können,

mit der Maßgabe, daß wenigestens einer der Substitunten $R^2$, $R^3$, $R^4$, $R^5$ und $R^6$ von Wasserstoff verschieden ist.

Die neuen O-Alkoxyalkyl-thionophosphonsäureester der Formel (I) zeichnen sich durch hohe Wirksamkeit gegen tierische Schädlinge, wie Insekten, Akariden und Nematoden aus und weisen günstige Eigenschaften als Schädlingsbekämpfungsmittel auf.

Man erhält die neuen Verbindungen der Formel (I), wenn man O-Alkoxyalkyl-thionophosphonsäureesterhalogenide der Formel II

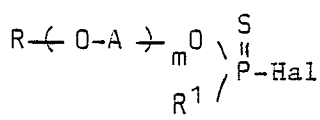

(II)

in welcher

R, A, m und $R^1$ die oben angegebene Bedeutung haben und Hal für Halogen (vorzugsweise Chlor und Brom, insbesondere Chlor) steht,

mit Phenolderivaten der Formel III

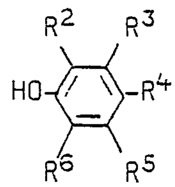

(III)

in welcher

$R^2$, $R^3$, $R^4$, $R^5$ und $R^6$ die oben angegebene Bedeutung haben,

oder mit deren Salzen gegebenenfalls in Gegenwart von Säurebindemitteln und gegebenenfalls in Gegenwart von Verdünnungsmitteln umsetzt.

Ueberraschenderweise zeigen die erfindungsgemäßen O-Alkoxyalkyl-thionophosphonsäureester der Formel (I) vorteilhaftere Eigenschaften als entsprechende aus dem Stand der Technik bekannte Verbindungen.

Ein besonderer Vorteil der erfindungsgemäßen Verbindungen liegt in ihrer systemischen Wirkungsweise: Sie können sowohl als blattsyste-

misch wirksame Insektizide und Akarizide als auchals wurzelsystemisch wirksame Bodeninsektizide und Nematizide verwendet werden.

Im gegebenenfalls substituierten Alkyl R, $R^1$, $R^3$, $R^4$ und $R^7$ steht Alkyl für geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n- und iso-Propyl sowie n-, iso-, sec- und tert-Butyl genannt. Methyl und Ethyl werden als Alkylreste besonders bevorzugt.

Halogen steht für Fluor, Chlor, Brom oder Iod, vorzugsweise für Fluor, Chlor oder Brom. Soweit Halogen Substituent am Phenylrest ist ($R^2$ bis $R^6$) steht es insbesondere für Chlor; soweit Halogen Substituent an einem Alkylrest ist (an R, $R^1$, $R^3$, $R^4$ und $R^7$) steht es insbesondere für Fluor oder Chlor.

Alkyl R, $R^1$, $R^3$, $R^4$ und $R^7$ kann durch einen oder mehrere, gleiche oder verschiedene Reste, vorzugsweise 1- bis 3-fach, substituiert sein. Als Beispiele für Substituenten seien Alkoxy- und Alkylthioreste mit vorzugsweise 1-4 Kohlenstoffatomen, Halogen, wie Fluor, Chlor, Brom und Jod, Cyano und Nitro genannt. Es werden die Halogensubstituenten wie oben angegeben bevorzugt.

Alkandiyl (Alkylen) steht für geradkettiges oder verzweigtes Alkandiyl mit vorzugsweise 2 bis 5, insbesondere 2 bis 3 Kohlenstoffatomen.

Beispielhaft seien Ethan-1,2-diyl ('Ethylen'), Propan-1,2-diyl und Propan-1,3-diyl genannt.

m steht für 1, 2 oder 3, vorzugsweise für 1 oder 2, insbesondere für 1.

n steht für 0, 1 oder 2, vorzugsweise für 0 oder 2, insbesondere für 0.

Die Erfindung betrifft vorzugsweise Verbindungen der Formel (I), in welcher

R für gegebenenfalls halogen-substituiertes geradkettiges oder verzweigtes Alkyl mit 1 bis 4 Kohlenstoffatomen steht,

A für geradkettiges oder verzweigtes Alkandiyl (Alkylen) mit 2 bis 5 Kohlenstoffatomen steht,

m für 1, 2 oder 3 steht,

$R^1$ für gegebenenfalls durch Fluor oder Chlor substituiertes geradkettiges oder verzweigtes Alkyl mit 1 bis 4 Kohlenstoffatomen steht,

$R^2$ für Wasserstoff, Fluor, Chlor, Brom oder Nitro steht,

$R^3$ für Wasserstoff, Fluor, Chlor, Brom, Nitro, Methyl, Ethyl oder Trifluormethyl steht,

$R^4$ für Wasserstoff, Fluor, Chlor, Brom, Methyl, Ethyl, Trifluormethyl steht und - für den Fall, daß $R^1$ für Methyl steht - auch für Nitro oder den Rest $-S(O)_nR^7$ steht, worin n für Null, 1 oder 2 steht und $R^7$ für Methyl, Ethyl oder Trifluormethyl steht und

$R^5$ und $R^6$ unabhängig voneinander für Wasserstoff und - für den Fall, daß $R^4$ für Halogen steht- auch für Fluor, Chlor oder Brom stehen können, mit der Maßgabe, daß wenigstens einer der Substituenten $R^2$, $R^3$, $R^4$, $R^5$ und $R^6$ von Wasserstoff verschieden ist und - für den Fall, daß $R^3$ oder $R^4$ für einen Alkylrest steht - wenigstens ein weiterer Substituent ($R^2$, $R^5$ und/oder $R^6$) von Wasserstoff verschieden ist.

Die Erfindung betrifft insbesondere Verbindungen der Formel (I), in welcher

R für geradkettiges oder verzweigtes Alkyl mit 1 bis 4 Kohlenstoffatomen steht,

A für geradkettiges oder verzweigtes Alkandiyl (Alkylen) mit 2 bis 3 Kohlenstoffatomen steht,

m für 1 oder 2 steht,

$R^1$ für Methyl steht,

$R^2$ für Wasserstoff steht,

$R^3$ für Wasserstoff oder Methyl steht,

$R^4$ für den Rest $-S(O)_nR^7$ steht, worin n für Null, 1 oder 2 steht und $R^7$ für Methyl steht, sowie

$R^5$ und $R^6$ für Wasserstoff stehen.

Als ganz besonders bevorzugte Verbindung der Formel (I) sei O-Methoxyethyl-O-(4-methylthio-phenyl)-thionomethanphosphonsäureester genannt ($R = CH_3$; $A = C_2H_4$; $m = 1$; $R^1 = CH_3$; $R^2$, $R^3$, $R^5$ und $R^6$ = H; $R^4 = SCH_3$).

Verwendet man beim erfindungsgemäßen Herstellungsverfahren für die Verbindungen der Formel (I) als Ausgangsstoffe beispielsweise O-Methoxyethyl-thionomethanphosphonsäureesterchlorid und 3-Methyl-4-methylsulfonyl-phenol, so kann der Reaktionsablauf durch folgendes Formelschema skizziert werden:

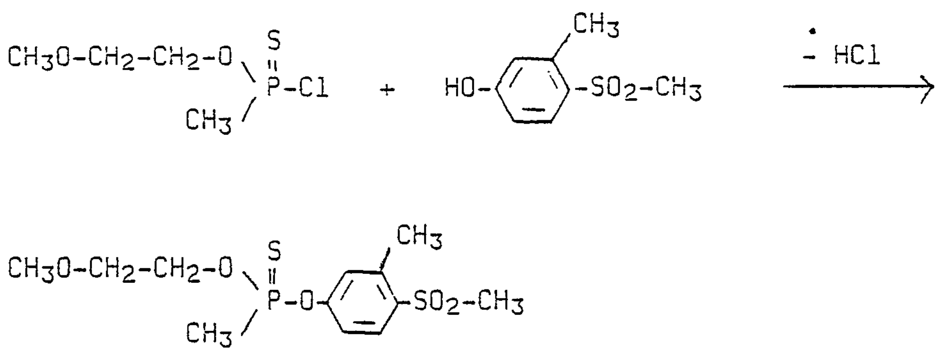


Die als Ausgangsstoffe zu verwendenden O-Alkoxyalkylthionophosphonsäureesterhalogenide sind durch die Formel (II) definiert. In dieser Formel haben R, A, m und $R^1$ die gleichen Bedeutungen, wie sie bei Formel (I) angegeben sind.

Als Beispiele für die Verbindungen der Formel (II) seien genannt: O-Methoxyethyl-, O-Ethoxyethyl-, O-n-Propoxyethyl-, O-iso-Propoxyethyl-, O-n -Butoxyethyl-, O-iso-Butoxyethyl-, O-(2-Methoxy-1-methylethyl)-, O-(2-Ethoxy-1-methyl-ethyl)-, O-( 2-n-Propoxy-1-methyl-ethyl)-, O-(2-iso-propoxy-1-methyl-ethyl)-, O-(2-n-Butoxy-1-methyl-ethyl)-, O-(2-iso-Butoxy-1-methyl-ethyl)-, O-(3-Methoxy-propyl)-, O-(3-Ethoxypropyl)-, O-(3-n-Propoxy-propyl)-, O-(3-iso-Propoxy-propyl)-, O-(3-n-Butoxy-propyl)-, O-(3-iso-Butoxy-propyl)-, O-Ethoxyethoxy-ethyl -, O-Methoxyethoxyethyl-, O-n-Propoxyethoxyethyl-, O-iso-Propoxyethoxyethyl-, O-n-Butoxyethoxyethyl- und O-iso-Butoxyethoxyethyl- -thionomethan-, -thionoethan-, -thionopropan- und -thionobutan-phosphonsäureesterchlorid.

Die O-Alkoxy-alkylthionophosphonsäureesterhalogenide der Formel (II) sind bekannt und/oder können nach an sich bekannten Verfahren hergestellt werden (vgl. AT-PS 283 051 und JP-PS 74 021 067).

Man erhält die Verbindungen der Formel (II) (Hal = Cl) durch Umsetzung von Thiophosphonsäuredichloriden der Formel IV

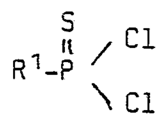

(IV)

in welcher

$R^1$ die oben angegebene Bedeutung hat,

mit Alkoxyalkanolen der Formel V

$$R(O\text{-}A)_{m}OH \qquad (V)$$

in welcher

R, A und m die oben angegebenen Bedeutungen haben,

gegebenenfalls in Gegenwart von Säurebindemitteln, wie z.B. Pyridin oder Collidin, und gegebenenfalls in Gegenwart von Verdünnungsmitteln wie z.B. Toluol oder Xylol, bei Temperaturen zwischen lo und 5o°C.

Thiophosphonsäuredichloride der Formel (IV) sind bereits bekannt (vergleiche Houben-Weyl, Methoden der organischen Chemie, 4. Aufl. Band 12/1, S. 552-557, Georg Thieme Verlag, Stuttgart 1963).

Als Beispiele für die Verbindungen der Formel (IV) seien genannt: Methanthiophosphonsäuredichlorid, Ethanthiophosphonsäuredichlorid, Propanthiophosphonsäuredichlorid und Butanthiophosphonsäuredichlorid.

Alkoxyalkanole der Formel (V) sind ebenfalls bekannt. Als Beispiele seien genannt:

Methoxyethanol, Ethoxyethanol, n-Propoxyethanol, iso-Propoxyethanol, n-Butoxyethanol, iso-Butoxyethanol, 2-Methoxy-1-methyl-ethanol, 2-Ethoxy-1-methyl-ethanol, 2-n-Propoxy-1-methyl-ethanol, 2-iso-Propoxy-1-methyl-ethanol, 2-n-Butoxy-1-methyl-ethanol, 2-iso-Butoxy-1-methyl-ethanol, 3-Methoxy-propanol, 3-Ethoxy-propanol, 3-n-Propoxy-propanol, 3-iso-Propoxy-propanol, 3-n-Butoxy-propanol, 3-iso-Butoxy-propanol, Ethoxy-ethoxyethanol, Methoxyethoxyethanol, n-Propoxyethoxyethanol, iso-Propoxyethoxyethanol, n-Butoxyethoxyethanol und iso-Butoxyethoxyethanol.

Die weiter als Ausgangsstoffe zu verwendenden Phenolderivate sind durch die Formel (III) definiert. In dieser Formel haben $R^2$, $R^3$, $R^4$, $R^5$ und $R^6$ die gleichen Bedeutungen, wie sie oben in Formel (I) angegeben sind.

Als Beispiele für die Verbindungen der Formel (III) seien genannt: 2-Chlor-phenol, 4-Chlor-phenol, 2,3-Dichlor-phenol, 2,4-Dichlor-phenol, 3,4-Dichlor-phenol, 2,4,5-Trichlor-phenol, 3,4,5-Trichlor-phenol, 2-Nitro-phenol, 3-Nitro-phenol, 4-Nitro-phenol, 2-Chlor-4-nitro-phenol, 3-Chlor-4-nitro-phenol, 4-Chlor-2-nitro-phenol, 4-Chlor-3-nitro-phenol, 2-Nitro-4-methyl-phenol, 3-Methyl-4-nitro-phenol, 4-Methylthio-phenol, 4-Ethylthio-phenol, 4-Methylsulfinyl-phenol, 4-Ethylsulfinyl-phenol, 4-Methylsulfonyl-phenol, 4-Ethylsulfonylphenol, 4-Trifluormethylthiophenol, 4-Trifluormethylsulfinyl-phenol, 4-Trifluormethylsulfonyl-phenol, 3-Methyl-4-methylthio-phenol, 3-Methyl-4-methylsulfinyl-phenol und 3-Methyl-4-methylsulfonyl-phenol.

Die Phenolderivate der Formel (III) sind bekannt.

Das Verfahren zur Herstellung von Verbindungen der Formel (I) wird vorzugsweise unter Verwendung von Verdünnungsmitteln durchgeführt.

0070443

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methyl-ethyl-, Methyl-isopropyl- und Methyl-isobutylketon, Ester, wie Essigsäuremethylester und -ethylester, Nitrile, wie z.B. Acetonitril und Propionitril, Amide, wie z.B. Dimethylformamid, Dimethylacetamid und N-methyl-pyrrolidon, sowie Dimethylsulfoxid, Tetramethylensulfon und Hexamethylphosphorsäuretriamid.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin. Es können auch die mit diesen Basen gebildeten Phenolsalze eingesetzt werden.
Die Reaktionstemperatur kann innerhalb eines größeren Bereichs variiert werden. Im allgemeinen arbeitet man zwischen 0 und loo°C, vorzugsweise bei 2o bis 6o°C.

Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Ueberschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt, und das Reaktionsgemisch wird mehrere Stunden bei der erforderlichen Temperatur gerührt. Dann wird gegebenenfalls mit Wasser verdünnt, ein mit Wasser praktisch nicht mischbares organisches Lösungsmittel, wie z.B. Toluol, dazugegeben und nach Durchschütteln die organische Phase wie üblich durch Waschen, Trocknen und Abdestillieren des Lösungsmittels aufgearbeitet.

Die neuen Verbindungen fallen in Form von Oelen an, die sich zum Teil nicht unzersetzt destillieren lassen, jedoch durch sogenanntes 'Andestillieren', d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Wirkstoffe (Verbindungen der Formel I) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.,
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma frugiperda, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis Atomaria spp., Oryzaephilus surinamensis, Anthonomus spp.,

Sitophilus spp., Otiorrhynchus sulcatus, Cosmopolites sordidus, Ceuthorrhynchus assimilis, Hypera postica, Dermestes spp. Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp. Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci ,

Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen

sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische

Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe könnne in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Be-

reichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

In den folgenden Beispielen, welche die Wirksamkeit der erfindungsgemäßen Verbindungen erläutern sollen, wurde vergleichend mit den Handelspräparaten Fenthion und/oder Methyl-Parathion (vgl. Pflanzenschutz und Schädlingsbekämpfung, Georg Thieme-Verlag, Stuttgart, 1977, Seiten 35 und 36) geprüft:

Beispiel A

Tetranychus-Test (resistent)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigten z.B. bei einer Konzentration von 0,1 % nach 2 Tagen die Verbindungen der Herstellungsbeispiele 1, 3, 5, 6, 8, 12, 14, 17, 24, 29, 30 und 32 eine Abtötung von 90 bis 100 % während Methyl-Parathion und Fenthion keine Abtötung (0 %) ergaben.

Beispiel B

Laphygma-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. bei einer Konzentration 0,001 % nach 3 Tagen die Verbindungen der Herstellungsbeispiele 16, 20, 22, 29 und 30 eine Abtötung von 80-100 % während Fenthion keine Abtötung (0 %) ergab.

0070443

Beispiel C

Grenzkonzentrations-Test / Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z.B. bei einer Konzentration von 20 ppm

0070443

die Verbindungen der Herstellungsbeispiele 1, 2, 3, 5, 6, 8, 12, 13, 17 und 18 eine Abtötung von 100 %, während Methyl-Parathion keine Abtötung ergab.

Beispiel D

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z.B. bei einer Konzentration von 20 ppm

die Verbindungen der Herstellungsbeispiele 1, 2, 3, 5, 6, 11, 12, 13, 17 und 18 eine Abtötung von 100 %, während Methyl-Parathion keine Abtötung (0 %) ergab.

Beispiel E

Grenzkonzentrations-Test

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigten bei einer beispielhaften Konzentration von 20 ppm z.B. die Verbindungen der Herstellungsbeispiele 1, 2, 3, 6, 10, 11 und 16 eine Abtötung von 100 %, während Methyl-Parathion keine Abtötung (0 %) ergab.

Die Herstellung der neuen Verbindungen der Formel I soll durch die folgenden Herstellungsbeispiele erläutert werden.

Beispiel 1

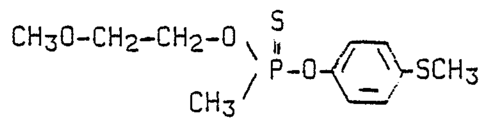

95 g (o,5 Mol) O-Methoxyethyl-methanthionophosphonsäureesterchlorid werden innerhalb von 3o Minuten zu einer gut gerührten Anschlämmung von 7o g (o,5 Mol) 4-Methylthio-phenol und 75 g Kaliumcarbonat in 5oo ml Acetonitril tropfenweise gegeben. Das Reaktionsgemisch wird ca. 3 Stunden bei 5o bis 55°C gerührt, nach Abkühlen in Wasser gegossen, mit Toluol verdünnt; nach Durchschütteln wird die organische Phase abgetrennt, zweimal mit Wasser gewaschen, über Natriumsulfat getrocknet, filtriert und vom Filtrat wird das Lösungsmittel unter vermindertem Druck abdestilliert. Der Rückstand wird im Hochvakuum andestilliert. Man erhält 123 g (84% der Theorie) O-Methoxyethyl-O-(4-methylthio-phenyl)-methanthionophosphonsäureester vom Siedepunkt 65°C/o,ool mbar (Brechungsindex $n_{D}^{2o}$: 1,5629).

Analog Beispiel 1 werden die in der nachstehenden Tabelle aufgeführten Verbindungen der Formel (I) hergestellt.

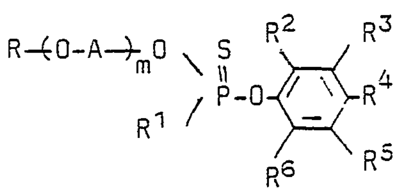

(I)

| Beispiel Nr. | R | A | m | $R^1$ | $R^2$ | $R^3$ | $R^4$ | $R^5$ | $R^6$ | Brechungsindex bzw. Siedepunkt (°C) |
|---|---|---|---|---|---|---|---|---|---|---|
| 2 | $CH_3$ | $-CH_2-CH_2-$ | 2 | $CH_3$ | H | H | $SCH_3$ | H | H | $n_D^{22}$:1,5587 |
| 3 | $CH_3$ | $-CH_2-CH_2-$ | 2 | $CH_3$ | H | $CH_3$ | $SCH_3$ | H | H | $n_D^{22}$:1,5549 |
| 4 | $C_2H_5$ | $-CH_2-CH_2-$ | 2 | $CH_3$ | H | H | $SCH_3$ | H | H | $n_D^{22}$:1,5519 |
| 5 | $C_2H_5$ | $-CH_2-CH_2-$ | 1 | $CH_3$ | H | H | $SCH_3$ | H | H | $n_D^{24}$:1,5619 |
| 6 | $CH_3$ | $-CH_2-CH_2-$ | 1 | $CH_3$ | H | $CH_3$ | $SCH_3$ | H | H | $n_D^{24}$:1,571o<br>9o°C/o,ool mbar |
| 7 | $n-C_4H_9$ | $-CH_2-CH_2-$ | 1 | $CH_3$ | H | H | $SCH_3$ | H | H | $n_D^{24}$: 1,5492 |
| 8 | $iso-C_3H_7$ | $-CH_2-CH_2-$ | 1 | $CH_3$ | H | $CH_3$ | $SCH_3$ | H | H | $n_D^{24}$: 1,5523 |
| 9 | $n-C_4H_9$ | $-CH_2-CH_2-$ | 2 | $CH_3$ | H | H | $SCH_3$ | H | H | $n_D^{24}$:1,5411 |
| 1o | $n-C_4H_9$ | $-CH_2-CH_2-$ | 2 | $CH_3$ | H | $CH_3$ | $SCH_3$ | H | H | $n_D^{24}$: 1,5297 |
| 11 | $C_2H_5$ | $-CH_2-CH_2-$ | 2 | $CH_3$ | H | $CH_3$ | $SCH_3$ | H | H | $n_D^{24}$: 1,5416 |
| 12 | $C_2H_5$ | $-CH_2-CH_2-$ | 1 | $CH_3$ | H | $CH_3$ | $SCH_3$ | H | H | $n_D^{24}$: 1,553o |

| Bei-spiel Nr. | R | A | m | $R^1$ | $R^2$ | $R^3$ | $R^4$ | $R^5$ | $R^6$ | Brechungsindex; Siedepunkt (°C) |
|---|---|---|---|---|---|---|---|---|---|---|
| 13 | iso-$C_3H_7$ | $-CH_2-CH_2-$ | 1 | $CH_3$ | H | H | $SCH_3$ | H | H | $n_D^{24}$: 1,54o4 |
| 14 | n-$C_4H_9$ | $-CH_2-CH_2-$ | 1 | $CH_3$ | H | $CH_3$ | $SCH_3$ | H | H | $n_D^{21}$: 1,54o1 |
| 15 | $C_2H_5$ | $-CH_2-CH_2-$ | 3 | $CH_3$ | H | H | $SCH_3$ | H | H | $n_D^{22}$: 1,5432 |
| 16 | $CH_3$ | $-CH_2-CH_2-$ | 1 | $CH_3$ | H | Cl | Cl | H | H | $n_D^{21}$: 1,555o |
| 17 | $CH_3$ | $-CH_2-CH_2-$ | 1 | $CH_3$ | H | H | $SOCH_3$ | H | H | $n_D^{20}$: 1,56o6<br>65°C/o,oo1 mbar |
| 18 | $CH_3$ | $-CH_2-CH_2-$ | 1 | $CH_3$ | H | H | $SO_2CH_3$ | H | H | $n_D^{20}$: 1,54o9<br>95°C/o,o1 mbar |
| 19 | $CH_3$ | $-CH_2-CH_2-$ | 1 | $CH_3$ | H | $NO_2$ | Cl | H | H | $n_D^{20}$: 1,5477<br>1oo°C/o,oo1 mbar |
| 20 | $CH_3$ | $-CH_2-CH_2-$ | 1 | $CH_3$ | H | H | $NO_2$ | H | H | $n_D^{20}$:1,5526<br>1oo°C/o,oo1 mbar |

| Beispiel Nr. | R | A | m | $R^1$ | $R^2$ | $R^3$ | $R^4$ | $R^5$ | $R^6$ | Brechungsindex; Siedepunkt (°C) |
|---|---|---|---|---|---|---|---|---|---|---|
| 21 | $CH_3$ | $-CH_2-CH_2-$ | 1 | $CH_3$ | H | $NO_2$ | H | H | H | $n_D^{20}$: 1,55o8<br>9o°C/o,ool mbar |
| 22 | $CH_3$ | $-CH_2-CH_2-$ | 1 | $CH_3$ | H | $CH_3$ | $NO_2$ | H | H | $n_D^{22}$: 1,5545 |
| 23 | $CH_3$ | $-CH_2-CH_2-$ | 1 | $CH_3$ | Cl | H | $NO_2$ | H | H | $n_D^{22}$: 1,5667 |
| 24 | $CH_3$ | $-CH_2-CH_2-CH_2-$ | 1 | $CH_3$ | H | H | $SCH_3$ | H | H | $n_D^{22}$: 1,5629 |
| 25 | $CH_3$ | $-CH_2-CH_2-CH_2-$ | 1 | $CH_3$ | H | $CH_3$ | $SCH_3$ | H | H | $n_D^{22}$: 1,5687 |
| 26 | $CH_3$ | $-CH_2-CH_2-$ | 1 | $CH_3$ | Cl | H | Cl | H | H | |
| 27 | $CH_3$ | $-CH_2-CH_2-$ | 1 | $CH_3$ | Cl | Cl | H | H | H | |

| Beispiel Nr. | R | A | m | $R^1$ | $R^2$ | $R^3$ | $R^4$ | $R^5$ | $R^6$ | Brechungsindex; Schmelzpunkt (°C) |
|---|---|---|---|---|---|---|---|---|---|---|
| 28 | $CH_3$ | $-CH_2-CH(CH_3)-$ | 1 | $CH_3$ | H | H | $SCH_3$ | H | H | $n_D^{22}$: 1,5782 |
| 29 | $CH_3$ | $-CH_2-CH_2-$ | 1 | $CH_3$ | H | Cl | Cl | Cl | H | 65-67 |
| 30 | $CH_3$ | $-CH_2-CH_2-$ | 1 | $CH_3$ | Cl | H | Cl | Cl | H | $n_D^{22}$: 1,5623 |
| 31 | $CH_3$ | $-CH_2-CH_2-$ | 1 | $CH_3$ | $NO_2$ | H | $CH_3$ | H | H | $n_D^{20}$: 1,5532 |
| 32 | iso-$C_3H_7$ | $-CH_2-CH_2-$ | 1 | $CH_3$ | H | Cl | Cl | H | H | |
| 33 | $C_2H_5$ | $-CH_2-CH_2-$ | 1 | $CH_3$ | H | Cl | Cl | H | H | |
| 34 | n-$C_4H_9$ | $-CH_2-CH_2-$ | 1 | $CH_3$ | H | Cl | Cl | H | H | |
| 35 | $C_2H_5$ | $-CH_2-CH_2-$ | 3 | $CH_3$ | H | H | $SCH_3$ | H | H | $n_D^{20}$: 1,5350 |
| 36 | $C_2H_5$ | $-CH_2-CH_2-$ | 3 | $CH_3$ | H | $CH_3$ | $SCH_3$ | H | H | $n_D^{20}$: 1,5333 |

Beispiel zur Herstellung von Ausgangsverbindungen der Formel (II) (Hal = Cl):

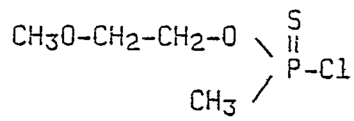

Eine Mischung aus 122 g Collidin und 76 g 2-Methoxy-ethanol wird innerhalb einer Stunde zu einer gut gerührten Lösung von 149 g Methanthiophosphonsäuredichlorid in 6oo ml Toluol bei einer Temperatur zwischen 3o und 35°C tropfenweise gegeben. Das Reaktionsgemisch wird über Nacht gerührt, mit Wasser, verdünnter Salzsäure und noch viermal mit Wasser gewaschen, über Natriumsulfat getrocknet. Das Toluol wird abgezogen und der Rückstand im Vakuum destilliert. Man erhält 131 g (69% der Theorie) O-Methoxy-ethyl-methanthionophosphonsäureesterchlorid vom Siedepunkt 94°C/4 mbar.

Patentansprüche

1. Thionophosphonsäureester der Formel I

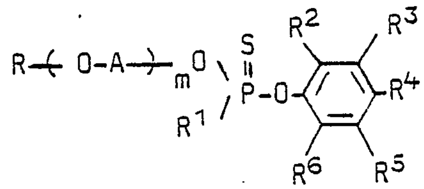

(I)

in welcher

R für gegebenenfalls substituiertes geradkettiges oder verzweigtes Alkyl steht,

A für geradkettiges oder verzweigtes Alkandiyl (Alkylen) steht,

m für 1, 2 oder 3 steht,

$R^1$ für gegebenenfalls substituiertes geradkettiges oder verzweigtes Alkyl steht,

$R^2$ für Wasserstoff, Halogen oder Nitro steht,

$R^3$ für Wasserstoff, Halogen, Nitro oder gegebenenfalls substituiertes Alkyl steht,

$R^4$ für Wasserstoff, Halogen, gegebenenfalls substituiertes Alkyl steht, und - für den Fall, daß $R^1$ für Methyl steht - auch für Nitro oder den Rest $-S(O)_nR^7$ steht, worin n für Null, 1 oder 2 steht und $R^7$ für gegebenenfalls substituiertes Alkyl steht und

$R^5$ und $R^6$ unabhängig voneinander für Wasserstoff und - für den Fall daß $R^4$ für Halogen steht - auch für Halogen stehen können,

mit der Maßgabe, daß wenigestens einer der Substitunten $R^2$, $R^3$, $R^4$, $R^5$ und $R^6$ von Wasserstoff verschieden ist.

2. Thionophosphonsäureester der Formel I gemäß Anspruch 1, in welcher

R für gegebenenfalls halogen-substituiertes geradkettiges oder verzweigtes Alkyl mit 1 bis 4 Kohlenstoffatomen steht,

A für geradkettiges oder verzweigtes Alkandiyl (Alkylen) mit 2 bis 5 Kohlenstoffatomen steht,

m für 1, 2 oder 3 steht,

$R^1$ für gegebenenfalls durch Fluor oder Chlor substituiertes geradkettiges oder verzweigtes Alkyl mit 1 bis 4 Kohlenstoffatomen steht,

$R^2$ für Wasserstoff, Fluor, Chlor, Brom oder Nitro steht,

$R^3$ für Wasserstoff, Fluor, Chlor, Brom, Nitro, Methyl, Ethyl oder Trifluormethyl steht,

$R^4$ für Wasserstoff, Fluor, Chlor, Brom, Methyl, Ethyl, Trifluormethyl steht und - für den Fall, daß $R^1$ für Methyl steht - auch für Nitro oder den Rest $-S(O)_nR^7$ steht, worin n für Null, 1 oder 2 steht und $R^7$ für Methyl, Ethyl oder Trifluormethyl steht und

$R^5$ und $R^6$ unabhängig voneinander für Wasserstoff und - für den Fall, daß $R^4$ für Halogen steht- auch für Fluor, Chlor oder Brom

stehen können, mit der Maßgabe, daß wenigstens einer der Substituenten $R^2$, $R^3$, $R^4$, $R^5$ und $R^6$ von Wasserstoff verschieden ist und - für den Fall, daß $R^3$ oder $R^4$ für einen Alkylrest steht - wenigstens ein weiterer Substituent ($R^2$, $R^5$ und/oder $R^6$) von Wasserstoff verschieden ist.

3. Thionophosphonsäureester der Formel I gemäß Anspruch 1, in welchen

R für geradkettiges oder verzweigtes Alkyl mit 1 bis 4 Kohlenstoffatomen steht,

A für geradkettiges oder verzweigtes Alkandiyl (Alkylen) mit 2 bis 3 Kohlenstoffatomen steht,

m für 1 oder 2 steht,

$R^1$ für Methyl steht,

$R^2$ für Wasserstoff steht,

$R^3$ für Wasserstoff oder Methyl steht,

$R^4$ für den Rest $-S(O)_nR^7$ steht, worin n für Null, 1 oder 2 steht und $R^7$ für Methyl steht, sowie

$R^5$ und $R^6$ für Wasserstoff stehen.

4. Thionophosphonsäureester der Formel

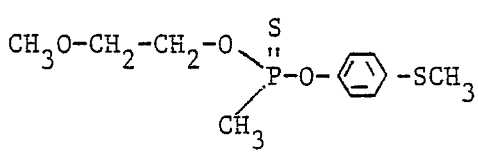

5. Thionophosphonsäureester der Formel

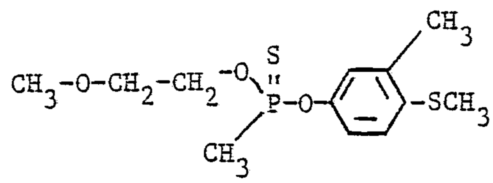

6. Verfahren zur Herstellung von Thionophosphonsäureestern der Formel I

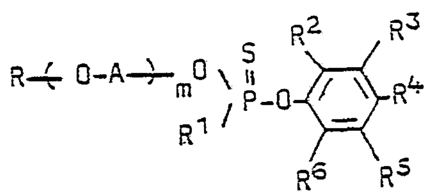

(I)

in welcher

R für gegebenenfalls halogen-substituiertes geradkettiges oder verzweigtes Alkyl mit 1 bis 4 Kohlenstoffatomen steht,

A für geradkettiges oder verzweigtes Alkandiyl (Alkylen) mit 2 bis 5 Kohlenstoffatomen steht,

m für 1, 2 oder 3 steht,

$R^1$ für gegebenenfalls durch Fluor oder Chlor substituiertes geradkettiges oder verzweigtes Alkyl mit 1 bis 4 Kohlenstoffatomen steht,

$R^2$ für Wasserstoff, Fluor, Chlor, Brom oder Nitro steht,

$R^3$ für Wasserstoff, Fluor, Chlor, Brom, Nitro, Methyl, Ethyl oder Trifluormethyl steht,

$R^4$ für Wasserstoff, Halogen, gegebenenfalls substituiertes Alkyl steht und - für den Fall, daß $R^1$ für Methyl steht - auch für Nitro oder den Rest $-S(O)_nR^7$ steht, worin n für Null, 1 oder 2 steht und $R^7$ für gegebenenfalls substituiertes Alkyl steht und

$R^5$ und $R^6$ unabhängig voneinander für Wasserstoff und - für den Fall, daß $R^4$ für Halogen steht - auch für Halogen stehen können,

mit der Maßgabe, daß wenigstens einer der Substituenten $R^2$, $R^3$, $R^4$, $R^5$ und $R^6$ von Wasserstoff verschieden ist,

dadurch gekennzeichnet, daß man Thionophosphonsäureester-halogenide der Formel

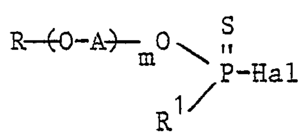

(II)

in welcher
R, A, m und $R^1$ die oben angegebene Bedeutung haben und Hal für Halogen steht,

mit Phenolderivaten der Formel III

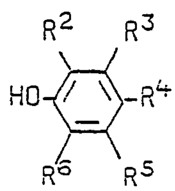

in welcher
$R^2$, $R^3$, $R^4$, $R^5$ und $R^6$ die oben angegebene Bedeutung haben,

oder mit deren Salzen gegebenenfalls in Gegenwart von Säurebindemitteln und gegebenenfalls in Gegenwart von Verdünnungsmitteln umsetzt.

7. Schädlingsbekämpfungsmittel, gekennzeichnet durch einen Gehalt an mindestens einem Thionophosphonsäureester der Formel I.

8. Verwendung von Thionophosphonsäureestern der Formel I zur Bekämpfung von Schädlingen, insbesondere Insekten, Spinnentieren und Nematoden.

9. Verfahren zur Bekämpfung von Schädlingen, dadurch gekennzeichnet, daß man Thionophosphonsäureester der Formel I auf die Schädlinge, vorzugsweise Insekten, Spinnentiere oder Nematoden und ihren Lebensraum einwirken läßt.

10. Verfahren zur Herstellung von Schädlingsbekämpfungsmitteln, dadurch gekennzeichnet, daß man Thionophosphonsäureester der Formel I mit Streckmitteln und/oder oberflächenaktiven Mitteln vermischt.

Europäisches Patentamt

# EUROPÄISCHER RECHERCHENBERICHT

0070443 Nummer der Anmeldung

EP 82 10 5969

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | CHEMICAL ABSTRACTS, Band 83, Nr.7, 18. August 1975, Seite 158, Zusammenfassung Nr.54618q, Columbus, Ohio (US) & JP - A - 74 24 653 (NIHON NOHYAKU CO., LTD.) (25. Juni 1974) * Zusammenfassung * --- | 1-10 | C 07 F 9/40<br>A 01 N 57/22 |
| X | CHEMICAL ABSTRACTS, Band 82, Nr.23, 9. Juni 1975, Seite 143, Zusammenfassung Nr.150534j, Columbus, Ohio (US) & JP - A - 74 21 067 (NIHON NOHYAKU CO., LTD.) (29. Mai 1974) * Zusammenfassung * --- | 1-10 | |
| Y | CHEMICAL ABSTRACTS, Band 82, Nr.23, 9. Juni 1975, Seite 143, Zusammenfassung Nr.150535k, Columbus, Ohio (US) & JP - A - 74 21 065 (NIHON NOHYAKU CO., LTD.) (29. Mai 1974) * Zusammenfassung * --- | 1-10 | RECHERCHIERTE SACHGEBIETE (Int. Cl. 3)<br>C 07 F 9/00 |
| Y | CHEMICAL ABSTRACTS, Band 85, Nr.17, 25. Oktober 1976, Seite 155, Zusammenfassung Nr.117986x, Columbus, Ohio (US) & JP - A - 75 10 781 (SUMITOMO CHEMICAL CO., LTD.) (24. April 1975) * Zusammenfassung * --- | 1-10 | |
| Y | DE-A-2 316 733 (BAYER AG.) * das ganze Dokument * ----- | 1-10 | |

Der vorliegende Recherchenbericht wurde für alle Patentansprüche erstellt.

| Recherchenort | Abschlußdatum der Recherche | Prüfer |
|---|---|---|
| DEN HAAG | 28-09-1982 | BESLIER L.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument